# EUROPEAN PATENT SPECIFICATION

(11) **EP 1 520 532 B1**
(45) Date of publication and mention of the grant of the patent: **28.11.2007**
(21) Application number: 04256053.2
(22) Date of filing: 30.09.2004
(51) Int. Cl.: A61B 17/11

(54) **Applier having automated release of surgical device**
Gerät zum Einbringen und automatischen Lösen einer chirurgischen Klammer
Applicateur ayant un système de libération automatique des clips chirurgicaux

(30) Priority: 30.09.2003 US 507800 P
(43) Date of publication of application: 06.04.2005
(73) Proprietor: ETHICON ENDO-SURGERY, INC., Cincinnati, Ohio 45242 (US)
(72) Inventor: Bumbalough, Timothy, Fullerton CA 92832 (US); Ruddocks, David, Mission Viejo CA 92692 (US)
(74) Representative: Tunstall, Christopher Stephen

(56) References cited:
- WO-A1-03/013370
- US-A- 5 540 712
- US-A- 5 617 874
- US-A- 6 007 544
- US-A1- 2002 082 627
- US-A1- 2003 120 292

## Description

### Field of the Invention

The present invention relates, in general, to appliers for surgical devices for surgically modifying organs and vessels. More particularly, it relates to appliers for anastomosis devices for joining two organs such as, for example, two separate lengths of small bowel to each other, a section of small bowel to the stomach, or the common bile duct to the duodeneum in a procedure called a choledochoduodenostomy.

### Background of the Invention

Creating an anastomosis, or the surgical formation of a passage between two normally distinct vessels, is a critical step of many surgical procedures. This is particularly true of gastric bypass procedures in which two portions of small intestine are joined together and another portion of small intestine is joined to the stomach of the patient. This is also true of surgery to alleviate blockage in the common bile duct by draining bile from the duct to the small intestine during surgery for pancreatic cancer.

For many anastomosis, surgeons use circular staplers, linear staplers, or manual sutures. However, to reduce incision size and to make the surgical process less technically demanding and time consuming, an expandable medical device that deforms to hold tissue portions together when the device is ejected from a constraining enclosure has been described. United States Application 2003/0120292 to Adrian Park et al, which is hereby incorporated herein by reference, describes such a device.

The expandable medical device disclosed in 2003/0120292 is constrained by a sheath to an advantageous small-diameter tubular shape. A surgeon applies the expandable medical device by maneuvering the sheath through the tissue portions requiring anastomosis, moving a nose piece distally away from the sheath, and ejecting the device from the applier. Ejecting the device removes the constraint on the device, allowing the device to assume a ring shape. The larger ends of the ring shape hold the two tissue portions together in an effective anastomosis.

A device such as that disclosed in 2003/0120292 may be made from a material such as superelastic nitinol. Devices made of superelastic nitinol can deform a great extent without yielding. When external forces tending to deform such devices are released, the devices return to their original geometry.

Applying an expandable medical device with the applier of 2003/0120292 requires two actions: separating the nosepiece and the sheath; and urging the expandable medical device distally relative to the sheath. Applicants have recognized the need for an applier that can operate with one control by the surgeon so that the surgeon controls one action while the applier performs the other action automatically at the proper time. Applicants have further recognized the need for an applier with a means to urge the device distally when the sheath is retracted, and a method for using the applier, so that the user needs only to use one control to activate the applier. More particularly, applicants have recognized the need for an instrument with a force element to urge the device distally when the sheath is retracted. This invention provides such an applier and for using it.

US 5,617,874 discloses a biopsy instrument having a stationary shaft secured to which is a stylet such that, once secured, the stylet does not move relative to the instrument. The stylet is telescopically received by a cannula which is mounted to a spring-loaded guide; the cannula and the guide move between charged and discharged positions. Also disclosed is a safety cap, which travels within slots formed in opposite sides of the cylindrical casing that houses the shaft, the guide and the spring. Retracting the safety cap moves the guide and the cannula from the discharged to the charged position where a release lever engages the guide. The safety cap covers the release lever to prevent accidental actuation of the instrument. The instrument of this doument is fired by sliding the safety cap forward and depressing the exposed release lever, which allows the spring to drive the cannula forward, toward the discharged position and over the stationary stylet, so that a tissue sample may be taken. Further, the biopsy instrument has a sealed chamber disposed behind the cannula.

WO 03/013370 is exemplary of the art. This document discloses a sutureless anastomosis system deployment concept. Specifically, anastomosis strain relief devices are disclosed which are disposed, at least partially, over an anastomosis bypass graft just proximal to an attachment site between a host vessel wall and the fitting. The strain relief devices provide additional support to the graft while preventing kinking of the graft, especially when it emanates at acute angles from the anastomosis site. Furthermore, the strain relief provides additional support to the graft during manipulations involved in inserting and attaching ends of the graft. The devices can have a variety of configurations, e.g., helical, zig-zag, etc., depending upon the desired functionality. The strain relief may also be either incorporated into the graft or placed exterior to the graft and bonded. Moreover, integrated fittings or collars may be incorporated into the strain relief to expand its functions.

### Summary of the Invention

In accordance with the present invention there is provided an applier for a surgical device that provides automated ejection of the device and gives the surgeon use of the applier with one operative control. The applier includes a handle, a tube movable relative to the handle, and an ejector shaft driven by a force element for ejecting the device. It may include a cap called a bullet nose or probe tip. The cap may have a tapered distal surface to ease entry into small otomies in tissue. Using a button or knob to move the tube automatically triggers the ejector shaft to eject the device when the tube is in the correct position.

### Brief Description of the Drawings

The novel features of the invention are set forth in the appended claims. The invention itself, however, together with further objects and advantages thereof, may best be understood by reference to the following description, taken in conjunction with the accompanying drawings in which:
Figure 1 is an isometric view of an applier according to an embodiment of the invention.
Figure 2 is an isometric view of the applier of Figure 1 with portions cut away for better visibility.
Figure 3 is a cross-sectional view of the applier of Figure 1 with an expandable medical device in the loaded position.
Figure 4 is a cross-sectional view of the applier of Figure 1 with the expandable medical device partially deployed.
Figure 5 is a cross-sectional view of the applier of Figure 1 with the expandable medical device fully deployed.
Figure 6 is an isometric view of an applier according to a second embodiment of the invention.
Figure 7A is an isometric view of a distal end of an applier showing a probe tip having a blunt surface.
Figure 7B is an isometric view of a distal end of an applier showing a probe tip having a fluted surface.
Figure 7C is an isometric view of a distal end of an applier showing a probe tip having a convex surface.
Figure 7D is an isometric view of a distal end of an applier showing a probe tip having a concave surface.
Figure 7E is an isometric view of a distal end of an applier showing a probe tip having an offset, swept, asymmetric surface.
Figure 7F is an isometric view of a distal end of an applier showing a probe tip having a spherical surface.

### Detailed Description of the Invention

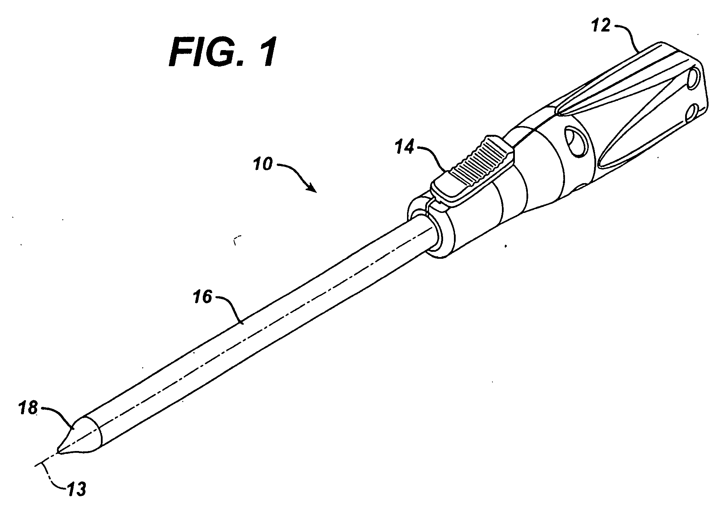

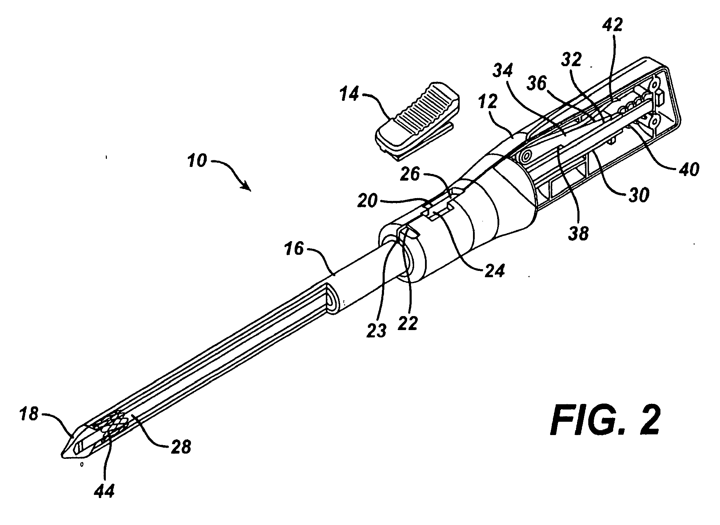

Figure 1 shows an embodiment of an applier 10 used to eject an expandable medical device 44 (Figure 2). Components of applier 10 visible in Figure 1 from the exterior include a frame, or handle 12, an actuator button 14, a device retainer, or tube 16, and a cap, called probe tip 18, at the distal end of tube 16.

In the embodiment of Figure 1, handle 12 comprises two handle halves. Handle 12 may be molded from engineering plastic and may be split into two halves to ease the assembly process of applier 10. The handle halves may be held together by a snap fit or by, for example, fasteners such as screws.

Tube 16 has a proximal portion located within handle 12, a distal portion extending from handle 12, and a longitudinal axis 13. Tube 16 can translate relative to handle 12 and rotate relative to handle 12 about longitudinal axis 13 of tube 16. Tube 16 may be made from metal or engineering plastic. At least a portion of tube 16 may be transparent or translucent to allow passage of light, either to utilize a light source internal to tube 16 to illuminate a surgical site, or to allow better visualization of internal components.

Actuator button 14 attaches to tube 16 by a press fit within a slot on tube 16, fasteners, or other commonly used attachment means. A surgeon uses actuator button 14 to translate tube 16 proximally towards handle 12 and to rotate tube 16 about longitudinal axis 13 of tube 16 as will be seen. Actuator button 14 can be made from engineering plastic and designed to deflect or flex. The deflection enables actuator button 14 to rock slightly proximally to distally about an axis perpendicular to longitudinal axis 13 of tube 16.

Probe tip 18 is fixed relative to handle 12. The distal end of probe tip 18 may be tapered as shown in Figure 1, or rounded, or have several shapes to facilitate entry through an opening in tissue or to assist in dilating tissue. The proximal end of probe tip 18 may also be chamfered, tapered, or rounded to help in operation of applier 10 as will be seen.

Figure 2 shows applier 10 of Figure 1 with portions removed for better visualization of components. Actuator button 14 is shown exploded away from applier 10 to reveal a roughly "Z"-shaped slot 20 in handle 12. Slot 20 contains a flaring portion 24 of slot 20. Slot 20 also contains an actuation portion 26, reachable by actuator button 14 by rotation about longitudinal axis 13 of tube 16. Handle 12 also possesses, revealed under exploded actuator button 14, a lock stop 22 and a rotation stop 23 at a distal end. Lock stop 22 and rotation stop 23 abut a boss raised on actuator button 14 when actuator button 14 is in a locked position.

Figure 2 further shows a probe tip shaft 28. Probe tip shaft 28 extends from supports within handle 12 distally to fix to probe tip 18, locating probe tip 18 relative to handle 12. Probe tip shaft 28 may attach to probe tip 18 by a threaded assembly. Handle 12 also contains the proximal portion of a pusher rod to eject expandable medical device 44, called ejector shaft 30. Ejector shaft 30 locates concentrically within tube 16, and extends distally from handle 12 to a point proximal of probe tip 18. Probe tip shaft 28 locates within the inner diameter of ejector shaft 30. At the proximal end of ejector shaft 30, ejector flange 32 flairs radially to a diameter larger than that of ejector shaft 30. Ejector flange 32 may consist of a washer pressed on a turned-down diameter of ejector shaft 30 and retained by a second washer, as shown in Figure 2. Alternately, flaring a proximal end of ejector shaft 30 in a formed or molded part may form ejector flange 32.

Shown distal of ejector shaft 30 in Figure 2 is a constraining member to prevent forward movement of ejector shaft 30, called stop latch 34. Stop latch 34 pins to handle 12 and rotates about the pinned junction. Stop latch 34 has a stop end 36 movable away and towards ejector flange 32. Stop latch 34 further has a cam surface 38. The proximal end of tube 16 contacts stop latch 34 along cam surface 38 to rotate stop latch 34.

Figure 2 further shows a force element in the form of ejector spring 40 compressed between a wall on handle 12 and a proximal side of ejector flange 32. Ejector spring 40 urges ejector shaft 30 distally. A second force element, latch spring 42, applies a force to rotate stop latch 34 so that stop end 36 bears against the distal side of ejector flange 32. In the embodiment shown, latch spring 42 is a leaf spring between a wall of handle 12 and stop latch 34. Latch spring 42 could also be, for example, a torsion spring having a torsional axis along the axis of rotation of stop latch 34.

Expandable medical device 44 locates near the distal end of applier 10 between the distal end of ejector shaft 30 and probe tip 18. Expandable medical device 44 may be, for example, an expandable medical device such as that described in United States Application number 2003/0120292. Such an expandable medical device will normally assume a ring-shaped appearance, but force applied by tube 16 will force the expandable medical device to assume a cylindrical shape.

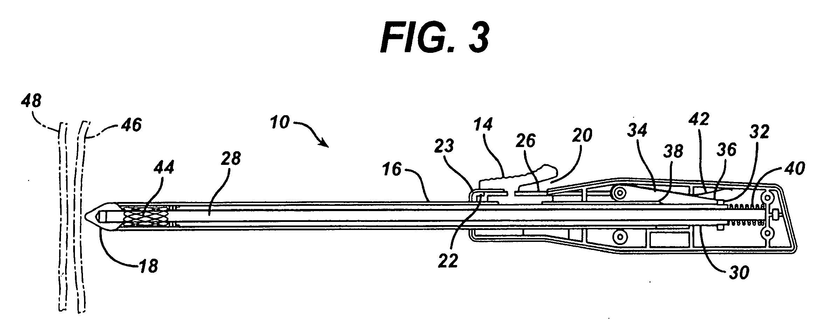

Figure 3 depicts a cross-section view of applier 10 of Figure 1, showing applier 10 containing expandable medical device 44. A surgeon uses applier 10 of Figure 1 to eject expandable medical device 44 to perform a surgical procedure, such as, for example, anastomosis of two body lumens. A surgeon grasps applier 10 and places it into a patient's body. The surgeon maneuvers applier 10 to a portion of the body near organs needing surgical treatment such as an anastomosis. The surgeon may, for example, first divide a section of small intestine as a part of a medical procedure such as a gastric bypass operation or as part of an operation to remove a section of intestine for cancer. The surgeon creates an otomy in a section of small intestine and extends applier 10 through the section of small intestine to a position where another otomy is desired. The surgeon then makes a second otomy in the wall of the same section of small intestine and a third otomy in another section of intestine to be anastomosed. Applier 10 can then extend through the second and third otomies in the two sections of small intestine. The walls carrying the second and third otomies can be shown as proximal tissue portion 46 and distal tissue portion 48. Attaching these two tissue portions creates an intestinal anastomosis when the tissue portions are lumens of intestine. After extending applier 10 through proximal tissue portion 46 and distal tissue portion 48, a surgeon can operate applier 10 to effect a medical procedure such as an anastomosis.

Applier 10 may be in a locked position. Such a locked position may be achieved by having actuator button 14 rotated slightly into the shorter section, or flaring portion 24 of Z-shaped slot 20 (Figure 2), and leaving actuator button 14 in the undeflected position. Actuator button 14, in an undeflected position, tilts distally slightly so that a boss raised on actuator button 14 contacts lock stop 22 and rotation stop 23. In the locked position, linear movement of actuator button 14 is precluded by lock stop 22 and rotational movement of actuator button 14 about the axis of tube 16 is precluded rotation stop 23. Slight pressure tilting actuator button 14 moves the boss away from lock stop 22 to permit movement of actuator button 14. This tilting or rocking motion is about an axis perpendicular to longitudinal axis 13 of tube 16.

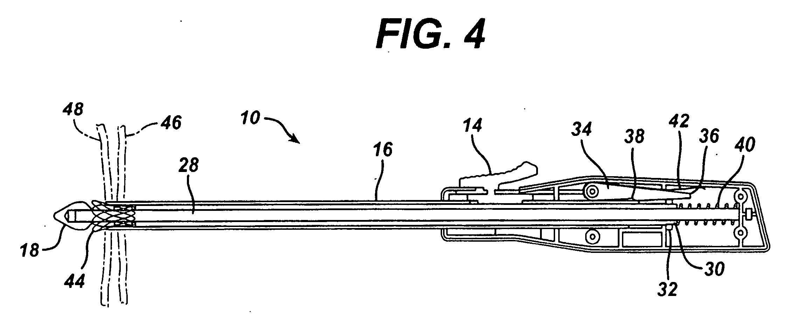

By urging actuator button 14 proximally, the surgeon begins to move tube 16 proximally towards stop latch 34. Initially, stop latch 34 is in a first position abutting ejector flange 32 of ejector shaft 30 to prevent distal movement of ejector shaft 30. A proximal portion of proximally moving tube 16 contacts cam surface 38 of stop latch 34 and begins to rotate stop latch 34 about the pivot point of stop latch 34. Latch spring 42 deflects while still applying a slight force tending to oppose the rotation of stop latch 34. Stop end 36 of stop latch 34 rotates away from ejector flange 32 to a second position permitting ejector spring 40 to move ejector shaft 30 distally. Ejector shaft 30 moves distally until ejector flange 32 reaches a boss within handle 12 that prevents further distal movement. Ejector shaft 30 drives expandable medical device 44 distally past the distal end of tube 16 to create the configuration shown in Figure 4. Expandable medical device 44 and ejector shaft 30 have moved relative to tube 16 a predetermined distance designed to best facilitate use of expandable medical device 44.

Actuator button 14 still remains in flaring portion 24 of slot 20, better depicted in Figure 2. Slight rotation of actuator button 14 about longitudinal axis 13 of tube 16 moves actuator button into the longer section, or actuation portion 26 of Z-shaped slot 20 (Figure 2). The rotation of actuator button 14 rotates tube 16 about longitudinal axis 13 of tube 16, and moves actuator button 14 away from a position distal to lock stop 22. Applier 10 then becomes ready to eject expandable medical device 44.

Figure 4 shows a partially ejected expandable medical device 44. Moving tube 16 proximally exposes a gap between tube 16 and probe tip 18. Expandable medical device 44, driven by ejector shaft 30, has moved towards probe tip 18. The tapered proximal end of probe tip 18 helps to flair expandable medical device 44 outwardly through the gap. The surgeon may move actuator button 14 slightly distally, moving tube 16 slightly distally against expandable medical device 44 to retain it in position. The surgeon may use applier 10 with the flared expandable medical device 44 as a tool to manipulate tissue. The surgeon may pull distal tissue portion 48 towards proximal tissue portion 46 using expandable medical device 44.

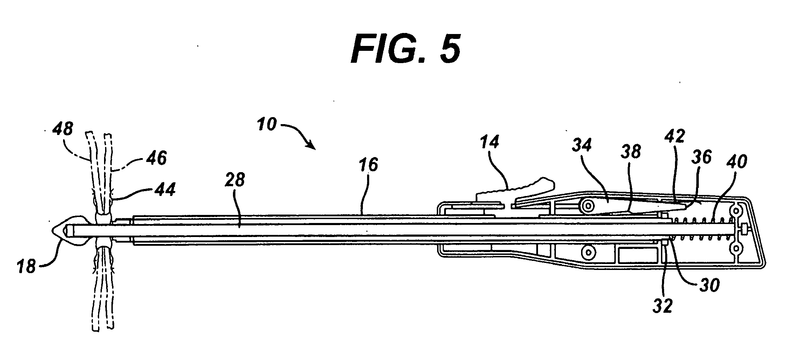

Figure 5 shows completely ejected expandable medical device 44. To eject expandable medical device 44, the surgeon moves actuator button 14 proximally, moving tube 16 proximally to release the proximal portion of expandable medical device 44. The distal end of ejector shaft 30 then becomes a restraint element to restrain proximal movement of expandable medical device 44 as tube 16 moves past expandable medical device 44. The embodiment of expandable medical device 44 shown in Figure 5 will expand, assume a ring shape, and force distal tissue portion 48 to proximal tissue portion 46 after the proximal portion of expandable medical device 44 is released from the confines of tube 16. In the embodiment of expandable medical device 44 shown in Figure 5, the inner diameter of expandable medical device 44 after application is larger than the outer diameter of probe tip 18.

The application of expandable medical device 44 is now complete. Because ejector spring 40 moves ejector shaft 30 distally, the surgeon needed only to move actuator button 14 proximally to eject expandable medical device 44. A second control to move ejector shaft 30 distally is not needed because proximal motion of tube 16 triggers, through stop latch 34, an automatic ejection of expandable medical device 44 by force applied by ejector spring 40.

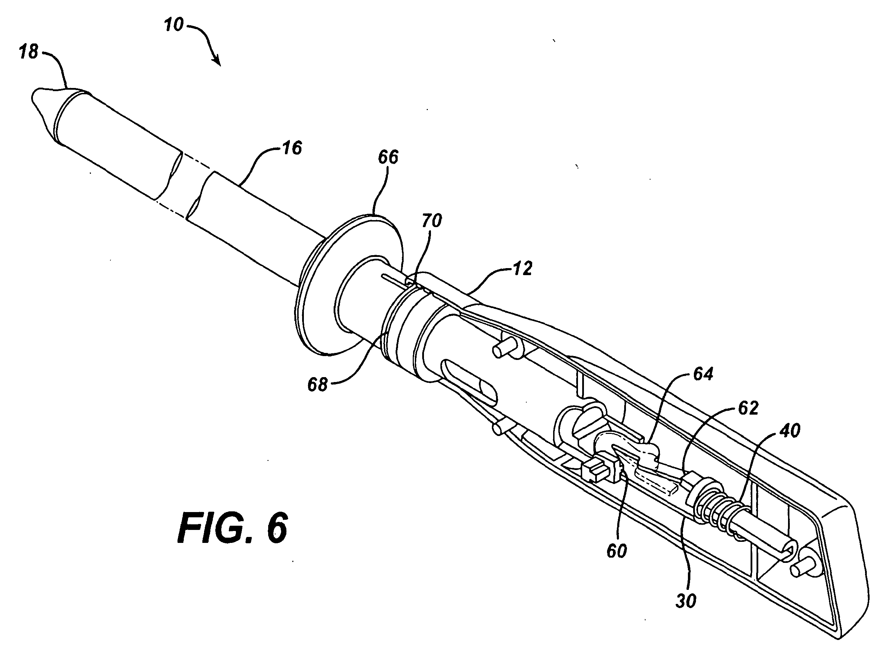

It will be recognized that equivalent structures may be substituted for the structures illustrated and described herein and that the described embodiment of the invention is not the only structure that may be employed to implement the claimed invention. One example of an equivalent structure that may be used to implement the present invention is shown in Figure 6. In the embodiment of Figure 6, "U"-shaped deflection beam 60 replaces stop latch 34 and latch spring 42. Deflection beam 60 is deflectable and has a restrainer stop 62 at a proximal end. Deflection beam 60 also has a restrainer cam surface 64.

Figure 6 further depicts a knob 66 attached to tube 16. Additionally, Figure 6 displays an o-ring 68 on knob 66 and a series of o-ring grooves 70 on handle 12, useful for tactile feedback as will be discussed.

When expandable medical device 44 is loaded into the applier 10 of Figure 6, ejector shaft 30 is held in the most proximal position by restrainer stop 62. As in the previous embodiment, ejector spring 40 applies a force urging ejector shaft 30 distally. Deflection beam 60, in a first, undeflected position, holds ejector shaft 30 from distal movement. Movement of tube 16 causes a proximal surface of tube 16 to contact restrainer cam surface 64, deflecting deflection beam 60 towards longitudinal axis 13 of tube 16 as shown in phantom in Figure 6. The deflection drives restrainer stop 62 to a second position, inwardly towards longitudinal axis 13 and away from the purchase that restrainer stop 62 has on ejector shaft 30. When ejector shaft 30 becomes released and no longer held by restrainer stop 62, ejector spring 40 moves ejector shaft 30 distally as in the previous embodiment.

A surgeon moves tube 16 proximally in the embodiment of Figure 6 by grasping knob 66. O-ring 68 moves through the series of o-ring grooves 70 to cause tactile feedback to the surgeon. O-ring 68 can cause tube 16 to detent at an advantageous position such as the position tube 16 is in at the point when deflection beam 60 releases ejector shaft 30.

As a further example of equivalent structures that may be anticipated, tube 16 and components contained within tube 16 may become long and flexible to maneuver through a long lumen such as a section of small bowel to effect an anastomosis through a long, flexible lumen. Such a long, flexible tube may be used laproscopically or endoscopically.

As a further example of an equivalent structure, applier 10 could have a long, rigid, curved tube, or a long, rigid, straight tube, and applier 10 could be placed through an obturator port and used laproscopically or endoscopically. Length and curvature become advantageous in endoscopic or laproscopic surgery, especially when performing a surgical procedure on a bariatric patient. In either a rigid or a flexible form of an applier 10, restriction of gas flow through the instrument becomes advantageous when maintenance of a pneumoperiteneum is desired. Such restriction may be accomplished by, for example, a seal or flow restrictor.

As a further example of an equivalent structure and method that may be used to implement the present invention, applier 10 may have a geometry small enough to be conveniently placed through the opening of a hand port used for hand-assisted laproscopic surgery, such as, for example, the Lap-Disk^{®} hand port sold by Ethicon Endo-Surgery in Cincinnati, Ohio. A surgeon using applier 10 through a hand port may use an endoscope through a secondary port for visualization, and may also maintain a pneumoperiteneum. The surgeon may also make use of trocars, graspers, cutters and other endoscopic instruments inserted through auxiliary ports to assist in grasping lumens or creating otomies in lumens to perform surgical procedures.

As a further example of an equivalent structure and method that may be used to implement the present invention, a long, rigid version of applier 10, or a long, flexible embodiment of applier 10 may be used through an auxiliary port while tissue is manipulated by the surgeon using a hand placed through a hand port.

It is also conceivable that the desired result of triggering an ejection of expandable medical device 44 simply by moving a device retainer could be achieved by other means. Applier 10 may also, instead of a compression spring shown as ejector spring 40 as a force element, use an extension spring attached between handle 12 and ejector shaft 30. Springs may be replaced by other means. Such means may include a small motor set to start driving the expandable medical device 44 to an ejected position when a moving device retainer trips a switch. Other means of achieving the same result will occur to those skilled in the art.

As another example of an equivalent structure, probe tip 18 and probe tip shaft 28 could have open distal and proximal ends and a lumen extending therethrough, so that a guidewire, fiber optic, or other useful surgical instrument may be placed through applier 10.

An embodiment of applier 10 may be attached to and utilized with computer-controlled robotic equipment. The robotic equipment enables a surgeon distant from the surgery site to use applier 10 to perform a procedure.

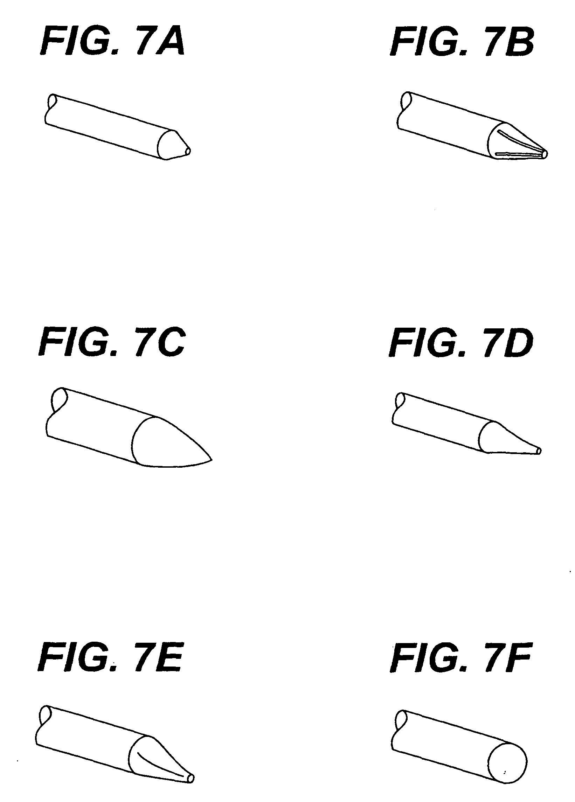

As other examples of equivalent structures, the surface of the distal taper on probe tip 18 may take many forms advantageous for various types of tissue manipulation, as illustrated in Figures 7A through 7F. Figure 7A represents a conical tipped nose that is blunted for low tissue trauma and for good visibility past the distal end. Figure 7B depicts a nose that is fluted to allow torque to be applied to tissue. Figure 7B depicts four flutes, although three or any other number of flutes may suffice. Figure 7C depicts a nose having a convex curve for rapid dilation of an otomy in a short space, while Figure 7D shows a nose having a concave surface for gentle dilation of friable tissue. An offset swept nose, shown in Figure 7E, may be used because of its asymmetry for better visibility to one side and may be used to assist in manipulation by using its asymmetry to minimally grasp tissue. Figure 7F shows a spherical nose to produce a short length for operation in limited space and to reduce the chance of tissue trauma. Combinations of these surfaces may also be advantageous, for example, a nose having a concave surface as depicted in Figure 7D may also posses flutes as depicted in Figure 7B. Probe tip 18 may possess a knife or a piercing element to create an otomy. Other combinations of shapes may occur to one skilled in the art.

While preferred embodiments of the present invention have been shown and described herein, it will be obvious to those skilled in the art that such embodiments are provided by way of example only. Numerous variations, changes, and substitutions will now occur to those skilled in the art without departing from the invention which is limited only by the scope of the appended claims.

## Claims

1. An applier (10) for a surgical procedure, said applier comprising:
a. a tube (16), being slidably movable proximally, having a distal end, a proximal end and a longitudinal axis therebetween;
b. a pusher rod (30) having distal and proximal ends, said pusher rod slidably disposed within said tube; and
c. an expandable medical device (44) having distal and proximal ends disposed within said distal end of said tube distal to said pusher rod;
**characterised in that**:
a means is provided for moving said pusher rod distally a predetermined distance upon moving said tube proximally so as to deploy said distal end of said device.

2. The applier of claim 1 wherein said applier includes a cap (18) disposed at its distal end, wherein said cap is distal to said distal end of said tube.

3. An applier according to Claim 1 wherein at least a portion of said tube is transparent.

4. An applier according to Claim 2 wherein said cap tapers distally to form a cone.

## Patentansprüche

1. Applikator (10) für eine chirurgische Prozedur, wobei der Applikator aufweist:
a. ein Rohr (16), das verschieblich proximal bewegbar ist, das ein distales Ende, ein proximales Ende und eine Längsachse zwischen diesen hat;
b. eine Schubstange (30) mit einem distalen und einem proximalen Ende, wobei die Schubstange verschieblich in dem Rohr angeordnet ist; und
c. eine aufweitbare medizinische Einheit (44) mit einem distalen und einem proximalen Ende, die innerhalb des distalen Endes des Rohrs distal zu der Schubstange angeordnet ist;
**dadurch gekennzeichnet, daß**:
eine Einrichtung zum Bewegen der Schubstange distal um eine vorbestimmte Entfernung nach dem proximalen Bewegen des Rohres, um so das distale Ende der Einheit zum Einsatz zu bringen, vorgesehen ist.

2. Applikator nach Anspruch 1, wobei der Applikator eine Kappe (18) umfaßt, die an seinem distalen Ende angeordnet ist, wobei die Kappe distal zu dem distalen Ende des Rohres ist.

3. Applikator nach Anspruch 1, bei dem wenigstens ein Teil des Rohres transparent ist.

4. Applikator nach Anspruch 2, bei dem die Kappe distal schräg verläuft, um einen Konus zu bilden.

## Revendications

1. Applicateur (10) pour une procédure chirurgicale, ledit applicateur comprenant:
a. un tube (16), mobile de façon coulissante et proximale, présentant une extrémité distale, une extrémité proximale et un axe longitudinal entre celles-ci ;
b. une tige-poussoir (30) comprenant des extrémités distale et proximale, ladite tige-poussoir étant disposée de manière coulissante à l'intérieur dudit tube ; et
c. un dispositif médical extensible (44) comprenant des extrémités distale et proximale disposées à l'intérieur de ladite extrémité distale dudit tube distal par rapport à ladite tige-poussoir ;
**caractérisé en ce que** :
■ un moyen est prévu pour déplacer ladite tige-poussoir de manière distale sur une distance prédéterminée lors du déplacement dudit tube de manière proximale de manière à déployer ladite extrémité distale dudit dispositif.

2. Applicateur selon la revendication 1 dans lequel ledit applicateur comporte un capuchon (18) disposé à son extrémité distale, dans lequel ledit capuchon est distal par rapport à ladite extrémité distale dudit tube.

3. Applicateur selon la revendication 1 dans lequel au moins une partie dudit tube est transparente.

4. Applicateur selon la revendication 2 dans lequel ledit capuchon se rétrécit de manière distale pour former un cône.
